# EUROPEAN PATENT APPLICATION

(11) **EP 1 806 158 A1**
(43) Date of publication of application: **11.07.2007**
(21) Application number: 05788397.7
(22) Date of filing: 27.09.2005
(51) Int. Cl.: A61M 16/04, A61M 1/00

(54) **TRACHEAL CANNULA**

(30) Priority: 27.09.2004 JP 2004280650; 04.04.2005 JP 2005108011
(71) Applicant: KOKEN CO., LTD., Toshima-ku, Tokyo 171-0031 (JP)
(72) Inventor: TOKUNAGA, Shuichi, 8790232 (JP); YAMAMOTO, Makoto, 8701123 (JP)
(74) Representative: Bertsch, Florian Oliver
(86) International application number: PCT/JP2005/017742
(87) International publication number: WO 2006/035769

(57) **Abstract**

A tracheal cannula of the invention includes a breathing path for supplying and discharging air formed between a basal end for connection with a breathing tube extending from an external artificial respirator and a distal end for insertion into a patient's trachea, and a trachea side opening formed at the distal end of the breathing path. Besides and along the breathing path, a suction path is provided for sucking phlegm accumulated in the patient's trachea. A phlegm suction opening is formed in a region of a wall of the suction path on the side of the inner wall of the trachea between the distal end of the breathing path and a cuff, the phlegm suction opening facing the inner wall of the trachea.

## Description

### Field of the Invention

The present invention relates to a tracheal cannula used for providing artificial respiration on a patient, and particularly to a tracheal cannula with which phlegm accumulated in the patient's trachea can effectively be sucked. The tracheal cannula according to the present invention relates not only to a tracheotomy tube inserted into the trachea through an opening formed in the patient's trachea in a tracheotomy operation, but also to a cannula for artificial respiration that is orally inserted into the trachea.

### Background of the Invention

A technique for providing artificial respiration with the use of an artificial respirator has been developed so far, in which a tracheal cannula is inserted into an incised trachea, and a breathing tube extended from the artificial respirator is communicated via an adapter with the tracheal cannula. Inside the tracheal cannula is formed a breathing path for supplying and discharging air. When providing artificial respiration, treatment of phlegm accumulated in the trachea and blocking up a respiratory tract is necessary. In a commonly used method for treating phlegm, a caretaker must disconnect the adapter from the tracheal cannula and then insert a suction catheter into the tracheal cannula through an opening (an opening on the side of the breathing tube) thereof to suck phlegm. With this treatment method, however, the caretaker is obliged to work any time day and night, thereby imposing an excessive burden upon the caretaker.

As a technique for solving such a problem, Japanese Utility Model Application Laid-open Publication No. S57-182449 (Patent Document 1) discloses a tracheal cannula provided with a pathway of small diameter formed in a sidewall (the left sidewall) of the tracheal cannula over the substantially entire length thereof, through which a suction catheter for sucking phlegm can be inserted. In this method, when sucking phlegm, a suction catheter is inserted along the pathway to suck phlegm accumulated in the trachea from a phlegm suction opening (a small opening) formed at the distal end of the suction catheter. Generally, the catheter is comprised of a tube (oval in cross-section), which is in the shape of a gradual circular arc having a large radius of curvature. In this example, the circumferential position of the phlegm suction opening formed in the suction catheter is on the opposite side of the inner wall on the trachea side (at a position of 180-degree phase difference with respect to the inner wall on the side of the trachea); more specifically, the phlegm suction opening is formed on the chest side of the patient lying on his/her back (see, FIGS. 6A and 6B in Patent Document 1). The trachea side opening of the breathing path and the trachea side opening of said pathway are located in the same plane perpendicular to the length direction of the breathing path (see, FIG. 2 in Patent Document 1). Further, along an outer circumference of the distal end of the tracheal cannula is provided a cuff (also termed as 'balloon') that expands and contracts in response to supply and discharge of air.
- Patent Document 1: Japanese Utility Model Application Laid-open Publication No. S57-182449

In using the tracheal cannula according to Patent Document 1, the distal end of the tracheal cannula is inserted first into an incised trachea and then expanded by infusing air into the cuff to fill a gap between the tracheal cannula and the inner wall of the trachea. The basal end of the tracheal cannula is then communicated with a breathing tube extending from an artificial respirator. When sucking phlegm, a suction catheter is inserted through the pathway on the sidewall of the tracheal cannula to suck phlegm accumulated in the trachea. In this case, treatment of phlegm in the left bronchus that required skills can be made easily with the use of the tracheal cannula according to Patent Document 1. This is because the pathway is provided on the sidewall of the tracheal cannula, and the tracheal cannula is gradually curved as a whole. In addition, the tracheal cannula disclosed in Patent Reference 1 may be used in such a way that phlegm in the trachea is sucked directly through the pathway as a phlegm suction path, instead of inserting a suction catheter through the pathway.

With respect to the tracheal cannula according to Patent Document 1, however, there are following problems:
(1) Suction of phlegm is delayed in the case where phlegm in the trachea is directly sucked through the pathway as a phlegm suction path instead of inserting the suction catheter through pathway. This is because the opening of the pathway on the trachea side (the phlegm suction opening) is located in the same plane (cross section) as the opening of the breathing path on the trachea side. In other words, as long as the opening of the pathway on the trachea side is not entirely covered with phlegm, suction is mainly made of air inside the respiratory tract or the lung, while substantially no phlegm can be sucked.
(2) Further, because the phlegm suction opening at the distal end of the suction catheter is located on the opposite side of the inner wall on the trachea side (on the inner side of the curvature) when fixed, suction of phlegm is not started until the substantially entire distal end of the suction catheter inserted into the trachea is covered with phlegm. Consequently, it is only after a certain amount of phlegm is accumulated in the trachea that the suction of phlegm is started.
(3) Moreover, if phlegm partly flows into the breathing path of the tracheal cannula during air discharge made by the artificial respirator, there is concern that smooth breathing may be disturbed because said part of phlegm having flowed into the breathing path cannot be removed.

### Disclosure of the Invention

It is an object of the present invention to provide a tracheal cannula that can suck phlegm promptly after it is produced.

It is another object of the present invention to provide a tracheal cannula that can reduce an amount of phlegm remaining in the trachea after suction of phlegm.

It is still another object of the present invention to provide a tracheal cannula that can remove phlegm having flowed into the breathing path.

In order to achieve these objects, a tracheal cannula according to the present invention comprises a basal end for connection with a breathing tube extending from an external artificial respirator and a distal end for insertion into a patient's trachea, a cuff provided circumferentially on a outer circumference of said distal end, the cuff expanding and contracting in response to supply and discharge of air, and a breathing path for supplying and discharging air provided between said basal end and said distal end, including an opening on the trachea side formed at the distal end of the breathing path; wherein a suction path for sucking phlegm accumulated in the patient's trachea is provided besides and along said breathing path, and a phlegm suction opening is formed in a region of a wall of said suction path on the side of an inner wall of the trachea between the distal end of said suction path and said cuff.

In using the tracheal cannula having the above configuration, the distal end of the tracheal cannula is inserted first into an incised trachea, and the cuff is then expanded by supplying air. By this, a gap between the tracheal cannula and the inner wall of the trachea is filled up. The breathing tube extending from the artificial respirator is then connected with the basal end of the tracheal cannula. Supply and discharge of air to or from the patient's trachea is made by the artificial respirator through the breathing path of the trachea cannula. The breathing path is always being connected with the artificial respirator via the breathing tube. Meanwhile, if phlegm is accumulated in the trachea, the phlegm is sucked away to the outside of the body with a negative pressure generated by operating a phlegm suction machine that is connected with the phlegm suction path via a suction tube. On this occasion, because the breathing path is always being connected with the artificial respirator, it is possible for the patient to breathe even during suction of phlegm.

In this case, in a region of a wall of the phlegm suction path on the side of the inner wall of the trachea is formed a phlegm suction opening between the distal end of the phlegm suction path and the cuff. More specifically, the phlegm suction opening is not formed perpendicular to the inner wall of the trachea, as is the case with the prior art, but formed so as to face the inner wall of the trachea. Consequently, even with a smaller amount of phlegm than needed when phlegm is sucked with the use of the suction catheter of the prior art, the substantially entire phlegm suction opening can be covered up. As a result, the phlegm in the trachea can be promptly sucked, and response of the tracheal cannula to emergence of phlegm can be improved. It has been found by the inventors for the first time that the advantageous effects as described above could be achieved by forming a phlegm suction opening on the wall of the phlegm suction path on the inner wall side of the trachea so as to face the inner wall of the trachea.

It is preferable with respect to the tracheal cannula according to the present invention that the suction opening of the phlegm suction path is located on the side of the cuff with respect to the opening formed on the trachea side of the distal end of the breathing path. In this case, a wall of the opening on the trachea side of the breathing path is formed between the distal end of the tracheal cannula and the phlegm suction opening. As a result, in sucking phlegm, even if a smallest part of phlegm accumulated in the trachea contacts the formed wall of the opening on the trachea side before the substantially entire phlegm suction opening is covered up, the phlegm will extend along the surface of that wall. As a matter of course, there is a part of phlegm then moving toward the phlegm suction opening. Therefore, the phlegm suction opening may more easily be covered up than in the case of the tracheal cannula of the prior art in which the opening of the breathing path on the trachea side and the opening of the pathway on the trachea side are located in a same plane perpendicular to the length direction of the breathing path. By this, phlegm can be sucked positively with a smaller amount of phlegm produced than that in the case of the prior art, and the phlegm in the trachea can be sucked promptly. Thus, the response of the tracheal cannula to emergence of phlegm can be improved.

With respect to the tracheal cannula of the present invention, it is preferable to form a further phlegm suction opening on a partition wall between the breathing path and the suction path so as to communicate the both paths to each other. In this case, even if suction of phlegm is made through the phlegm suction opening directly connecting the phlegm suction path and the trachea in such a condition that the opening is tightly appressed to the inner wall of the trachea (i.e., futile suction), that part of the inner wall of the trachea is not absorbed into the phlegm suction opening at the distal end of the cannula. This is because there is provided another phlegm suction opening on the partition wall of the phlegm suction path on the breathing path side, which functions as a relief valve. In sucking phlegm, the phlegm suction opening formed in the partition wall is covered up when phlegm flows into the suction path through the phlegm suction opening formed in the wall of the suction path on the side of the inner wall of the trachea, since a membrane of phlegm forms in the suction path. Therefore, the power of suction of phlegm through the phlegm suction opening on the side of the inner wall of the trachea is not to be decreased because of the phlegm suction opening formed in the partition wall. Further, in discharging air with the use of the artificial respirator, there may be a concern that the phlegm flows into the breathing path of the tracheal cannula to interfere with smooth breathing. However, since phlegm accumulated around the distal end of the breathing path of the tracheal cannula is sucked through the phlegm suction opening formed at the distal end of the phlegm suction path on the partition wall, the phlegm can readily be removed if ever the phlegm has flowed into the breathing path. Thus, there is practically no danger of interference of smooth breathing.

With respect to the tracheal cannula of the present invention, the distal end of the phlegm suction path may either be closed or left open so that it can be connected to the trachea. Further, a region of the partition wall between the breathing path and the suction path may end up before the distal end of the trachea cannula (between the basal end and the distal end) to form a communication space in which the distal ends of the breathing path and the suction path are communicated with each other and which, in turn, is to be communicated with the trachea.

Preferred embodiments of the present invention will be explained below in further detail with reference to the drawings.

### Brief Description of the Drawings

FIG. 1 is a perspective view on an artificial respiration system including the tracheal cannula of the present invention.
FIGS. 2a and 2b are schematic diagrams respectively showing a vertical section and a horizontal section of the tracheal cannula according to a first embodiment of the present invention.
FIG. 3 is also a schematic diagram illustrating a modified example of the tracheal cannula of the first embodiment.
FIGS. 4a and 4b are schematic diagrams respectively showing a vertical section and a horizontal section of a tracheal cannula according to a second embodiment of the present invention.
FIGS. 5a, 5b, 5c and 5d are schematic diagrams illustrating phlegm suction operations with the use of the tracheal cannula of the second embodiment.
FIG. 6 is a schematic diagram illustrating phlegm suction operations with the use of a trachea cannula according to a third embodiment of the present invention.
FIG. 7 is a schematic diagram illustrating phlegm suction operations with the use of a tracheal cannula according to a fourth embodiment of the present invention.
FIG. 8 is a schematic diagram illustrating phlegm suction operations with the use of a tracheal cannula according to a fifth embodiment of the present invention.

### Best Mode for Carrying Out the Invention

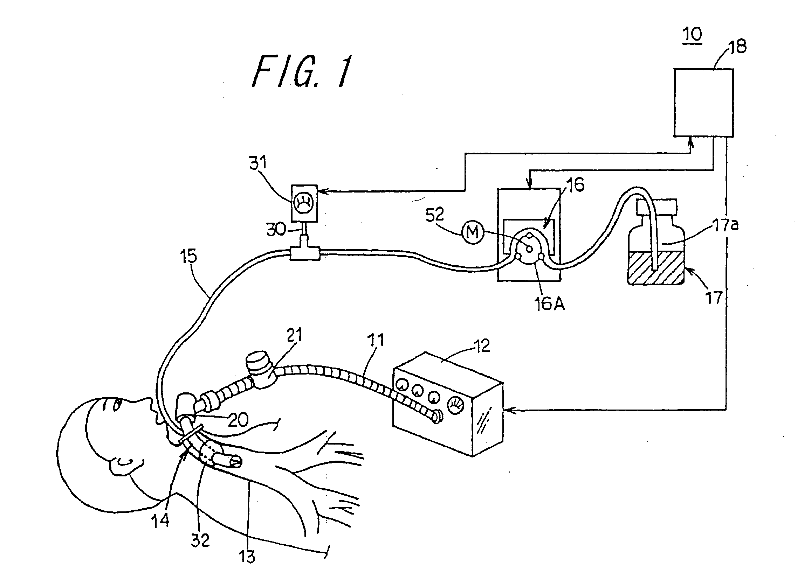

In FIG. 1 is shown an artificial respiration system represented as a whole by reference numeral 10, and applied with a tracheal cannula 14 according to a first embodiment of the present invention. The artificial respiration system 10 comprises an artificial respirator 12 communicated with a breathing tube 11 for supplying and discharging air, the above-mentioned tracheal cannula 14 which is to be inserted into a patient's incised trachea 13, a phlegm suction machine 16 communicated with a suction tube 15 for sucking phlegm accumulated in the trachea 13, and a phlegm collection bottle 17. The artificial respiration system 10 further comprises a control box 18 including a sequencer, timer, etc., for controlling an amount of inlet flow into the phlegm suction machine 16 by regulating a suction pressure of the phlegm suction machine 16, based on an inner pressure of the suction tube 15.

The artificial respirator 12 has a configuration known, *per se,* in which breathing is secured by alternately putting into operation a built-in suction machine and a compressor at regular time intervals. More specifically, the breathing tube 11 communicates a air supply/discharge port of the artificial respirator 12 with a mount (joint part) 20 provided at the basal end of the tracheal cannula 14. Further, on an intermediate portion of the breathing tube 11 is provided a breathing valve 21.

A main body 14A of the tracheal cannula 14 comprises a plastic tube having a predetermined length and being curved substantially in the shape of J, and can be inserted into a patient's trachea 13 through an opening having been formed by a tracheotomy operation on the trachea 13. The main body 14A of the tracheal cannula has a circular cross-section, an inner diameter thereof being 8 to 12 mm, for example, and a breathing path 14a is formed inside it, and the distal end of the main body 14A opens into the patient's trachea.

On an outer circumference of the distal end of the main body 14A of the tracheal cannula is provided an annular cuff 32 so as to be attached closely to the outer circumference of the main body 14A. The cuff expands and contracts in response to supply and discharge of air. The cuff 32 is a circular balloon comprising a synthetic resin sheet. On the outer circumference of the main body 14A of the tracheal cannula is fixed a fine tube 32a comprising a synthetic resin for supplying air into the cuff 32. At the basal end of the fine tube 32a is provided a small cylindrical adapter 33 having a valve. The cuff can be expanded by inserting a tip of a syringe into the adapter 33 to supply air through the fine tube 32a into the cuff 32.

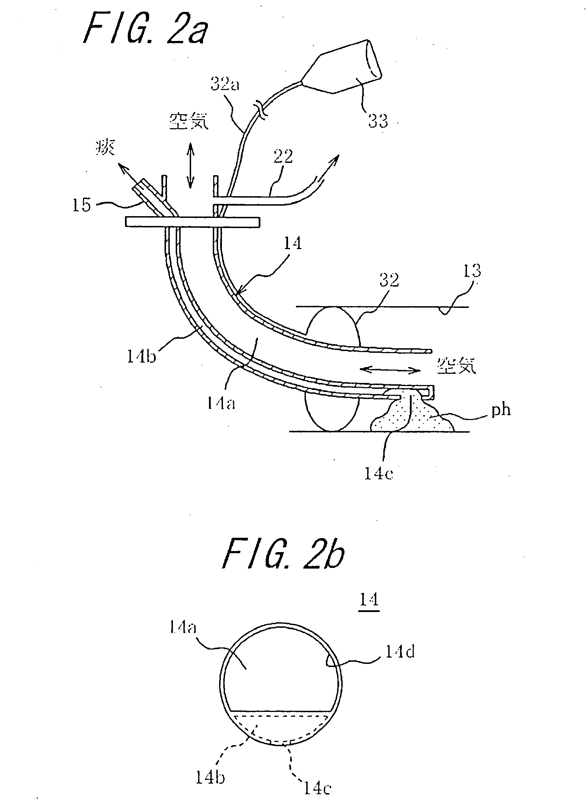

As shown in FIGS. 2a and 2b, inside the main body 14A of the tracheal cannula substantially in the shape of a cylinder is formed the breathing path 14a described above and also a suction path 14b on one side of this breathing path 14a. The both paths are separated by a partition wall 140. More specifically, the internal space of the main body 14A of the tracheal cannula is separated by the partition wall 140 into a breathing path 14a having a large sectional area and a suction path 14b substantially in the shape of a half-moon having a smaller sectional area. The phlegm suction path 14b is located, on the outer circumference of the breathing path 14a, on the side opposite to the center of the curvature of the outer circumferential wall of the main body 14A of the tracheal cannula curving substantially in the shape of J. In other words, when the tracheal cannula 14 is appropriately fixed on the patient lying on his/her back, the phlegm suction path 14b is located on the downside (on the back side) of the patient, along the J-curvature. It is also possible to thicken the tube wall of the main body 14A of the tracheal cannula to form a phlegm suction path 14b inside the tube. In this case, a region of the tube wall on the breathing path side will function as a partition wall that divides the breathing path 14a and the suction path 14b. At the distal end f the phlegm suction path 14b is formed, on the underside thereof, a phlegm suction opening 14c, for example in the shape of an oval.

Further, at the distal end of the breathing path 14a is formed an opening 14d on the trachea side. The opening 14d communicates the internal space of the trachea with the breathing tube 11 of the artificial respirator 12. The distal end of the phlegm suction path 14b is closed. Suction of phlegm into the phlegm suction path 14b is made through the phlegm suction opening 14c at the distal end thereof. The phlegm suction opening 14c is not confined to an oval shape, and it may also be in a slit-like shape having a predetermined width and length.

The basal end of the main body 14A of the tracheal cannula 14 is connected to the distal end of the suction tube 15 extending from the phlegm suction machine 16 and detouring around the mount 20. The outer diameter of the suction tube 15 is 4 mm, for example.

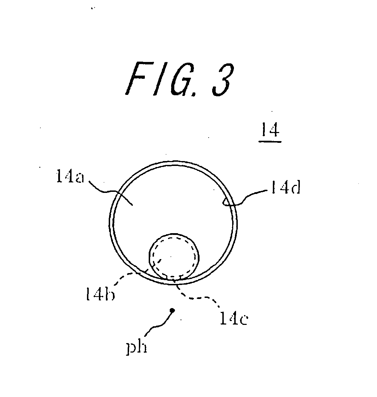

The phlegm suction path 14b is a passage way having a flat partition wall 140 separating the suction path from the breathing path 14a and having a cross-section perpendicular to the length direction thereof in the shape of a half-moon, as shown in an illustrated example in FIG. 2b. In FIG. 3 is shown a modified example, in which the cross-section of the suction path 14b is in a circular shape. The cross-section of the suction path 14b may also be in a different shape than those described above, and it may be in an oval shape, for example. In each case, the suction path 14b is formed along the curvature of the main body 14A of the tracheal cannula on the convex side of the curvature. This convex side of the curvature will come close or be appressed to the bottom side of the inner wall of the trachea when it is fixed on the trachea of the patient lying on his/her back, while the opposite (concave) side will draw away from the bottom side of the inner wall of the trachea. As described above, the cross-section of the breathing path 14a is larger than that of the suction path 14b. This will make it easier for the patient to breathe.

The suction tube 15 is an elongate elastic tube comprising a flexible synthetic resin, as shown in FIG. 1. On an upstream side (on the side of the tracheal cannula 14) of the suction tube 15 with respect to the intermediate portion thereof in the length direction, the suction tube 15 is connected to a short diverging tube 30, which is used when detecting a suction pressure of the phlegm suction machine 16. The diverging tube is, at the distal end thereof (at the upper end in FIG. 1), connected to a pressure sensor 31. An inner pressure of the suction path 14b (i.e. a phlegm suction pressure inside the suction path 14b) can be measured with the use of the pressure sensor 31.

The phlegm suction machine 16 sucks phlegm accumulated in the trachea 13 with a negative pressure generated inside it through the phlegm suction path 14b and the suction tube 15 and collects the phlegm into a collection bottle 17. As illustrated in FIG. 1, the phlegm suction machine 16 comprises a suitable pump such as a tube pump 16A.
The tube pump 16A comprises a rotor provided with three pressure rollers for locally pressing and blocking up the suction tube 15 comprising a synthetic resin having a predetermined flexibility, the pressure rollers being located on an outer circumference of the rotor in a protruding condition so as to distance themselves from each other in a circumferential direction, a pressure guide a provided at a predetermined distance from the outer circumferential surface of the rotor, a surface of the pressure guide facing the rotor (a tube pressure surface) being curved parallel to the outer circumferential surface of the rotor, and a rotation means for rotating the rotor. The rotation means of the rotors comprises an electric motor 52 and rotates the rotors along the tube pressure surface of the pressure guide. A rough indication of an amount of pressure feed (a pressure feed velocity) of the phlegm by means of the tube pump 16A is 50 to 200 cc/sec.

A control unit 18 controls the phlegm suction operation in the following manner. In normal suction of phlegm, the control unit 18 operates the tube pump 16A with a preset value (a normal suction pressure in phlegm suction), while it sends an operation command to the tube pump 16A to power up the pumping for a predetermined length of time if a value detected by the pressure sensor 31 exceeds the preset value. In other words, the tube pump 16 is operated regularly during a normal operation of the phlegm suction machine 16, while the output power of the tube pump 16A is boosted in unusual circumstances, for example one in which the suction tube 15 is blocked up by phlegm. In preparation for such unusual circumstances, an auxiliary pump controlled by the control unit 18, such as a diaphragm pump, may be provided additionally to the phlegm suction machine 16.

Operation of the automatic suction system 10 including the tracheal cannula 14 according to the first embodiment will be explained below.

First, the distal end of the main body 14A of the tracheal cannula 14 is inserted into a patient's incised trachea 13. In this case, the convex surface of the main body 14A of the tracheal cannula 14 having a predetermined radius of curvature is located on the under side so as to face the inner wall of the trachea. Next, air is supplied into the cuff 32 with a syringe to expand it. By this, a gap between the circumferential wall of the main body 14A of the tracheal cannula and the inner wall of the trachea is filled up with the cuff 32. Then, the breathing tube 11 extending from the artificial respirator 12 is connected to the basal end of the main body 14A of the tracheal cannula. The patient's breathing, i.e. supply/discharge of air to and from the patient's trachea 13 with the use of the artificial respirator 12, is made through the breathing path 14a in the main body 14A of the tracheal cannula via the breathing tube 11 and the mount 20, in a similar manner as in the prior art.

If phlegm ph is accumulated inside the patient's trachea, the tube pump 16A communicated with the phlegm suction path 14b via the suction tube 15 is brought into operation. As a result, the phlegm is sucked with a negative pressure generated in the pump 16A into the suction path 14b through the phlegm suction opening 14c to be discharged to the outside of the body through the suction path 14b and the suction tube 15. Since the breathing path 14a is always being communicated with the artificial respirator 12, it is always possible for the patient to breathe even during suction of phlegm. The phlegm suction operation by the tube pump 16A will be explained hereinafter.

The phlegm suction path 14b is closed at the distal end thereof. The phlegm suction opening 14c is located on the side of the cuff 32 with respect to the trachea side opening 14d of the breathing path 14a. More specifically, the phlegm suction opening 14c does not open perpendicularly toward the inner wall of the trachea, as is the case for the prior art, but in such a way as to face parallel to the inner wall of the trachea. The formed wall of the trachea side opening 14d of the breathing path 14a is thus exposed on the side of the distal end of the tracheal cannula 14 with respect to the phlegm suction opening 14c, As a result, in sucking phlegm, even if a smallest part of phlegm accumulated in the trachea contacts the formed wall of the opening on the trachea side before the substantially entire phlegm suction opening is covered up, the phlegm will extend along the surface of that wall. As a matter of course, there is a part of phlegm then moving toward the phlegm suction opening. Therefore, the phlegm suction opening may more easily be covered up than in the case of the tracheal cannula of the prior. By this, phlegm can be sucked positively with a smaller amount of phlegm produced than that in the case of the prior art. Thus, phlegm in the trachea 13 can be sucked promptly, to improve the response of the tracheal cannula to emergence of phlegm.

The phlegm suction operation with the use of the tube pump 15A will next be explained in detail. First, while the suction tube 15 is placed between the outer circumferential surface of the rotor and the tube pressure surface of the pressure guide, the rotor is rotated by means of the rotation means 52 in a direction of phlegm discharge. By this, the pressure roller is rotated in the direction of phlegm discharge along the tube pressing surface of the pressure guide, and the suction tube 15 is partly blocked up between the tube pressing surface of the pressure guide and the pressure roller. The position of blockage is continuously moved with rotation of the pressure roller in the direction of phlegm discharge. Consequently, the position of blockage in the suction tube 15 is gradually moved in the direction of phlegm discharge so as to generate a negative pressure on the suction side of the suction tube 15. Phlegm in the trachea 13 is sucked by this negative pressure, through the phlegm suction opening 14c and the suction path 14b into the suction tube 14. Then, the phlegm in the suction tube 15 is gradually fed toward the discharge side and eventually discharged from the suction tube 15. Thus, the phlegm suction machine 16 may be obtained in a simple manner and at low cost.

The inner pressure of the tube is not increased when the phlegm suction opening 14c of the phlegm suction path 14b is not blocked with phlegm. It is not until the phlegm suction opening 14c is covered up with phlegm that the suction pressure of the tube pump 16A starts to increase. Therefore, even if the cuff is deflated to make the phlegm suction opening 14c of the suction tube 15 fixed on the trachea 13 contact the inner wall of the trachea, there is substantially no danger of hurting the inner wall of the trachea because the suction pressure is not increased, preventing parts of the inner wall of the trachea from being sucked up into the suction path 14b. In addition, since the suction is made at a constant rate, it is possible to detect positively and promptly, with the use of the pressure sensor 31, whether or not there is phlegm.

In normal operation, the inner pressure of the suction tube 15 is increased unusually if such an excessive amount of phlegm that it cannot be handled by the tube pump 16A is generated or if the suction tube 15 is blocked up with phlegm of high viscosity. The increased pressure is detected by the pressure sensor 31, and a command for increasing the output power is sent from the control unit 18 to the tube pump 16A. As a result, it is possible to avoid unusual circumstances during suction of phlegm and to prevent an accident such as trachea obstruction.

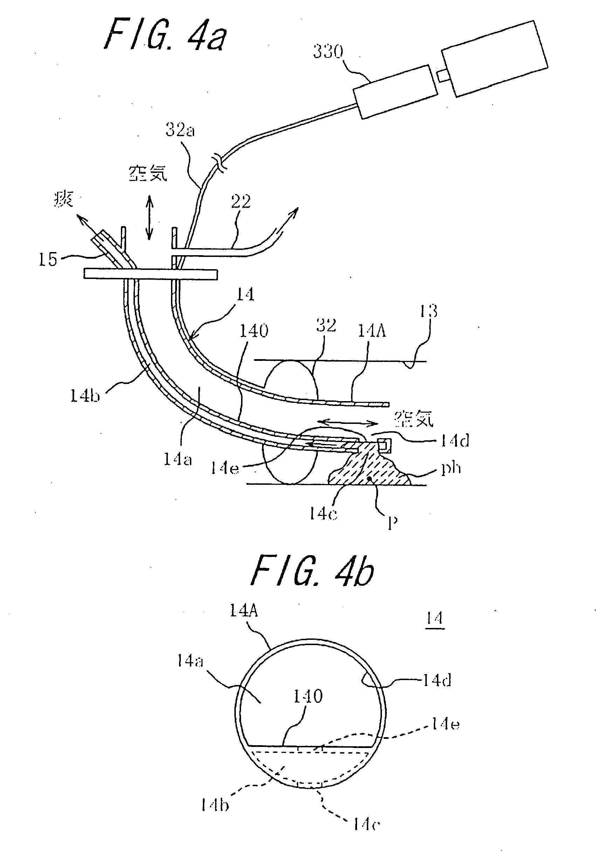

In FIGS. 4a and 4b is illustrated a tracheal cannula according to a second embodiment of the present invention. The tracheal cannula of the present embodiment is substantially of the same configuration as that of the first embodiment, except that an additional phlegm suction opening 14e having substantially the same size as that of the phlegm suction opening 14c is formed in the partition wall 140 on the top surface of the phlegm suction path 14b, i.e. on the opposite side of the phlegm suction opening 14c. The phlegm suction opening 14e opens up into and is communicated with the distal end of the breathing path 14a of the tracheal cannula 14.

When phlegm ph is accumulated in the patient's trachea 13, the tube pump 16A is operated in the same way as in the first embodiment. Consequently, the phlegm is sucked into the suction path 14b through the phlegm suction opening 14c, due to a negative pressure generated in the pump 16A, the phlegm being then discharged to the outside of the body through the suction path 14b and the suction tube 15. The phlegm is sucked into the suction path 14b through the both phlegm suction openings 14c and 14e. In other words, phlegm in the main body 14A of the tracheal cannula can also be sucked at once, as well as phlegm on the inner wall of the trachea 13.

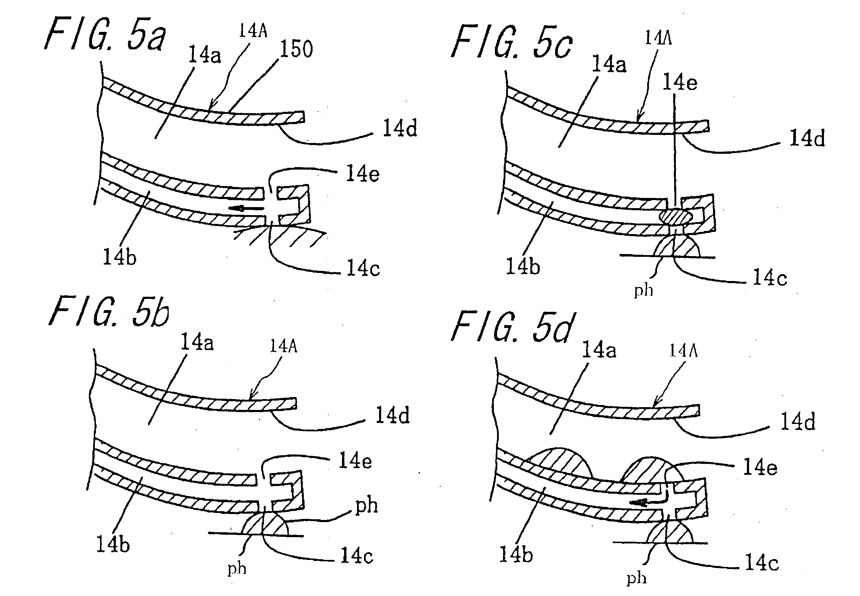

FIG. 5a shows a situation in which there is no phlegm ph in the trachea. In this case, while the phlegm suction opening 14c on the bottom side is appressed to the inner wall of the trachea, the phlegm suction opening 14e on the upper side is left open. Therefore, the phlegm suction opening 14c on the bottom side will not entirely be blocked up because the phlegm suction opening 14c sucks the inner wall of the trachea. Thus, it is possible to prevent the inner wall of the trachea to be sucked into the suction opening 14c.

FIGS. 5b and 5c show a situation in which there is phlegm ph accumulated in the trachea. The phlegm is sucked into the suction path 14b through the phlegm suction opening 14c. In this case, the phlegm suction opening 14e on the upper side is covered with a thin membrane. FIG. 5d illustrates a suction operation in a case where a piece of phlegm has flowed into the breathing path 14a of the tracheal cannula 14. As shown in FIG. 5d, the phlegm in the breathing path 14a is sucked through the phlegm suction opening 14e on the upper side. On this occasion, it is possible for the patient to breathe even during suction of phlegm because the breathing path 14a is always communicated with the artificial respirator 12.

In sucking phlegm, there is substantially no danger of a part of the inner wall of the trachea being sucked through the phlegm suction opening 14c, even if an idle suction is made while the phlegm suction opening 14c on the side of the inner wall of the trachea is appressed to the inner wall of the trachea. This is because there is provided another phlegm suction opening 14e on the wall of the phlegm suction path 14b on the side of the breathing path 14a. More specifically, since the other phlegm suction opening 14e functions as a relieve valve during suction of phlegm, there is not generated an excessively large negative pressure in the other phlegm suction opening 14e even if the phlegm suction opening 14c on the side of the inner wall of the trachea is appressed to the inner wall of the trachea.

In sucking phlegm ph, the phlegm suction opening 14e formed in the partition wall 140 on the side of the breathing path 14a of the phlegm suction path 14b will be covered up if phlegm has flowed into the suction path 14b, since a membrane of phlegm is formed (generated) along the inner circumferential surface of the distal end of the suction path 14b. As a result, there is substantially no danger of causing a significant decrease in the power of suction of phlegm because of the phlegm suction opening 14e formed in the wall on the side of the breathing path 14a of the phlegm suction path 14b (FIGS. 5b and 5c). Such advantageous effects were found by the inventors for the first time.

Furthermore, there may be concern that smooth breathing is interfered with if a phlegm flows into the breathing path 14a of the tracheal cannula 14 in discharging air by the artificial respirator 10. However, the phlegm in the breathing path 14a is sucked through the phlegm suction opening 14e formed in the wall of the main body of the cannula. Thus, there is little interference to smooth breathing by means of the breathing path 14a, even if phlegm has flowed into the breathing path 14a (see FIG. 5d).

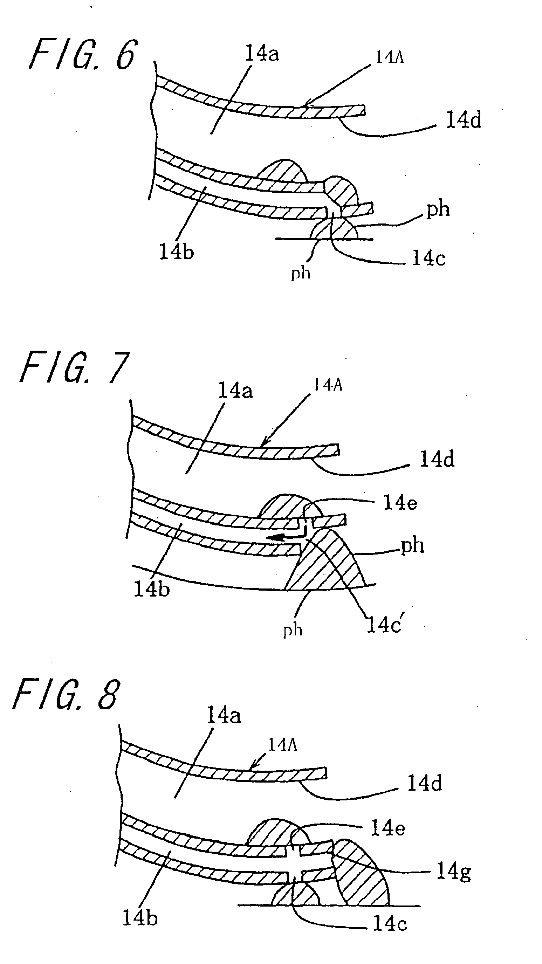

FIG. 6 shows a third embodiment of the tracheal cannula according to the present invention. In the present embodiment, the distal end of the phlegm suction path 14b of the tracheal cannula 14 is left open. Thus, the breathing path 14a and the suction path 14b divided from each other by a partition wall 140, which is shorter than the main body 14A of the cannula (i.e., the distal end of the partition wall 140 is cut away), are communicated with each other in the opened part at the distal end of the phlegm suction path 14b. This opened part is an equivalent of the phlegm suction opening 14e in the second embodiment, and even phlegm in the breathing path 14a of the main body of the tracheal cannula can be sucked through this opened part. Further, instead of this cutaway, a slit of a predetermined width may also be formed at the distal end of the partition wall 140 over a predetermined length. When the tracheal cannula is set, the breathing path 14a on the upper side and the phlegm suction path 14b on the bottom side are communicated with each other via this slit.

With the embodiment of FIG. 6 having such configuration, phlegm is sucked through the phlegm suction opening 14c on the bottom side, in the same manner as in the above embodiments. In addition, since there is formed a large cutaway, i.e. another phlegm suction opening on the upper side (equivalent to the phlegm suction opening 14e in the case of the second embodiment), or a slit at the distal end of the partition wall, phlegm in the breathing path 14a can be sucked from this opened part (i.e., the part where it is communicated with the distal end opening 14d of the main body 14A of the tracheal cannula). It is easier to form a slit at the distal end of the partition wall than to form a cutaway. Other configurations and operations are the same as those in the above-described embodiments.

In FIG. 7 is illustrated a tracheal cannula according to a fourth embodiment of the present invention. In this embodiment, a part of the distal end of the phlegm suction path 14b in the tracheal cannula 14 (a part of the bottom of the main body 14A of the cannula) is cutaway to form an opening. Although the breathing path 14a and the suction path 14b are separated by the partition wall 140, the both paths are communicated with each other through the phlegm suction opening 14e formed at the distal end of the partition wall 140. At the same time, on the bottom side of the distal end of the phlegm suction path 14b is formed an opening or an opened part, which is located below the distal end of the partition wall 140 so as to communicate the phlegm suction path 14b with the trachea. This opening or opened part 14c' functions as an equivalent of the phlegm suction path 14c in the above-described embodiments, so that phlegm outside the main body 14A of the cannula and on the bottom of the trachea can be sucked through this opening 14c'. The opening 14d at the distal end of the breathing path 14a is located on the side of the distal end in the length direction of the main body 14A of the cannula with respect to the opening 14c'.

With the embodiment of FIG. 7 having such configuration, while phlegm is sucked through the phlegm suction opening 14e in the same manner as in the above-described embodiments, there is formed a large cutaway, i.e. a phlegm suction opening 14c', on the lower side of the suction opening 14e, so that phlegm in the trachea can be sucked from this cutaway (i.e. the part where the suction path 14b is opened into the trachea which is located outside the distal end of the main body 14A of the tracheal cannula). Other configurations and operations are the same as those in the above-described embodiments.

In FIG. 8 is illustrated a tracheal cannula according to a fifth embodiment of the present invention. In this embodiment, the distal end of the phlegm suction path 14b of the tracheal cannula 14 is opened (14g) in the same plane as the distal end opening 14d the breathing path 14a. The breathing path 14a and the suction path 14b separated by the partition wall 140 are communicated with each other through a phlegm suction opening 14e formed at the distal end of the partition wall 140. At the same time, the phlegm suction path 14b is opened to the trachea at the distal end thereof through the opening 14g. Besides the distal end opening 14g, there is also formed a phlegm suction opening 14c, which is similar to those in the above-described embodiments, between the distal end of the main body 14A of the cannula and the cuff 32. Consequently, by generating a negative pressure in the suction path 14b with the use of the suction machine 16, the phlegm in the breathing path 14a can be sucked through the phlegm suction opening 14e, and phlegm on the bottom of the trachea outside the main body 14A of the tracheal cannula can also be sucked through the opening 14g and the phlegm suction opening 14c.

With the embodiment of FIG. 8 having such configuration, while the phlegm is sucked through the phlegm suction opening 14e in the same way as in the above-described embodiments, there is formed an distal end opening 14g in addition to the phlegm suction opening 14c located on the lower side of the phlegm suction opening 14e, so that phlegm in the trachea can also be sucked through this opening 14g. Thus, phlegm accumulated in the trachea can be sucked promptly. Other configurations and operations are the same as those in the above-described embodiments.

It will be understood from the foregoing description that, with the tracheal cannula of the present invention, it is possible to suck phlegm promptly after it is accumulated, to reduce an amount of phlegm remaining in the trachea after suction of phlegm, and to remove phlegm having been flowed into the breathing path. As a matter of course, the tracheal cannula according to the present invention can not only be applied to a tracheotomy tube, which is to be inserted into the trachea through an opening formed in the patient's trachea in a tracheotomy operation, but also to a cannula for artificial respiration, which is to be orally inserted into the trachea.

## Claims

1. A tracheal cannula comprising:
a basal end for connection with a breathing tube extending from an external artificial respirator, and a distal end for insertion into a patient's trachea,
a cuff provided circumferentially on a outer circumference of said distal end, the cuff expanding and contracting in response to supply and discharge of air, and
a breathing path for supplying and discharging air provided between said basal end and said distal end, and including a trachea side opening at the distal end of the breathing path;
wherein a suction path for sucking phlegm accumulated in the patient's trachea is provided besides and along said breathing path, and a phlegm suction opening is formed in a region of a wall of said suction path on the side of an inner wall of the trachea between the distal end of said suction path and said cuff.

2. The tracheal cannula according to claim 1, wherein said phlegm suction opening is located on the side of said cuff with respect to the trachea side opening formed at the distal end of said breathing path.

3. The tracheal cannula according to claim 1, wherein a further phlegm suction opening is formed in said partition wall, the phlegm suction opening communicating said breathing path with said suction path.

4. The tracheal cannula according to claim 1, wherein said suction path is closed at the distal end thereof.

5. The tracheal cannula according to claim 1, wherein said suction path is left open at the distal end thereof so as to be communicated with said trachea.

6. The tracheal cannula according to claim 1, wherein a part of said partition wall ends up before the distal end of said cannula so that distal parts of said breathing path and said suction path are communicated with each other to form a communication space, the communication space being in turn communicated with said trachea.
